# EUROPEAN PATENT APPLICATION

(11) **EP 1 348 335 A1**
(43) Date of publication of application: **01.10.2003**
(21) Application number: 02007129.6
(22) Date of filing: 28.03.2002
(51) Int. Cl.: A01K 67/027, C12N 5/10, C12N 5/16

(54) **Non-human transgenic mammals and their use as a disease model**

(71) Applicant: Boehringer Ingelheim International GmbH, 55216 Ingelheim (DE)
(72) Inventor: Fleischmann, Alexander, New York, NY 10027 (US); Wagner, Erwin, 1030 Wien (AT); Jochum, Wolfram, 55606 Kirn (DE); Radolf, Martin, 2392 Sulz im Wienerwald (AT)
(74) Representative: Laudien, Dieter, Dr.

(57) **Abstract**

A non-human mammal that carries germline mutations in both the *p53* and the *Fos* genes, methods for producing it and cell lines derived therefrom.

The *p53*/*Fos* double knockout animal, in particular a mouse, is useful as a rhabdomyosarcoma disease model.

## Description

### Field of the invention

The present invention is in the field of oncology, in particular in the field of animal cancer models.

### Introduction

Rhabdomyosarcoma is the most common soft-tissue sarcoma of childhood and accounts for 4-8 % of all pediatric malignancies ¹.
Rhabdomyosarcomas are histologically classified as embryonal, alveoar, or pleomorphic ². Although rhabdomyosarcomas display rhabdomyoblastic differentiation, the cell type of origin has not been identified yet. The molecular pathogenesis of human rhabdomyosarcoma is poorly understood. Most rhabdomyosarcoma of the alveolar subtype carry the characteristic t(2;13)(q35;q14) and t(1;13)(p36;q14) translocations which result in the formation of the chimeric PAX3-FKHR and PAX7-FKHR transcription factors ³⁻⁵. In addition, the *TP53* tumor suppressor gene, among others like MDM2, CDKN2A (p16^{INK4}) and CDK4 (reviewed in ⁶), is frequently mutated in sporadic embryonal rhabdomyosarcoma ^{7,8}. A function for the *TP53* pathway in rhabdomyosarcoma tumorigenesis is further indicated by the observation that rhabdomyosarcoma is the most common sarcoma type in patients with hereditary predisposition to cancer due to germ-line mutations in the *TP53* gene ⁹⁻¹¹. Rhabdomyosarcomas also develop in *p53* heterozygous and homozygous mutant mice, although with low penetrance and long latency ^{12,13}. Mutated *TP53* may function through an inhibition of MYOD function, thereby blocking differentiation ¹⁴, or by loss of the normal transcriptional repression of IGF-2 by wildtype TP53 ¹⁵.

Evading apoptosis is an important mechanism in the pathogenesis of rhabdomyosarcoma. Besides enhancing cellular proliferation and invasion ³³, the PAX3-FKHR fusion protein of alveloar rhabdomyosarcoma protects tumor cells against apoptosis ^{34,35}, most likely by induction of BCL-XL ³⁶, c-MET ^{37,38} and other regulators of myogenic differentiation ^{39,40}. Furthermore, IGF2 which has important functions in normal skeletal muscle development and acts as a survival factor in various tumor types, is overexpressed in the majority of human rhabdomyosarcomas ^{41,42}. Overexpression in rhabdomyosarcomas is achieved either by loss of the maternal IGF-2 allele, which is normally silenced by imprinting, and duplication of the paternal allele ^{43,44}, or by loss of imprinting of the maternal allele ⁴⁵. Recently, IGF2 overexpression has also been linked to inactivation of the tumor suppressor Patched *(Ptc),* the receptor of sonic hedgehog (*shh*). Mice heterozygous for a targeted mutation of the *Ptc* gene develop embryonal rhabdomyosarcoma with low incidence ⁴⁶. In these tumors, *Igf2* and the transcription factor *Gli-1*, a mediator of *Ptc* signaling, are overexpressed suggesting that Ptc negatively regulates lgf2 expression and genetic evidence indicates that rhabdomyosarcoma development in *Ptc1*+/- mice depends on *lgf2* gene expression ⁴⁷.

The Fos protein is a major component of the AP-1 transcription factor, which regulates various biological processes by converting extracellular signals into changes in the expression of specific target genes ¹⁶. Mice lacking Fos develop osteopetrosis due to the lack of osteoclasts, the resorbing cell type of the bone ¹⁷⁻¹⁹. Consistent with its original identification as the transforming activity of the mouse osteosarcoma viruses FBJ-MuSV and FRB-MuSV ²⁰, ectopic expression of Fos in embryonal stem cells and transgenic mice results in oncogenic transformation and the development of chondrosarcoma and osteosarcoma, respectively ^{21,22}. In addition, Fos mediates both pro- and anti-apoptotic signals and regulates invasive growth and angiogenesis during tumorigenesis ²³⁻²⁶

### Summary of the invention

While rhabdomyosarcoma is the most common soft-tissue sarcoma of childhood, animal models for rhabdomyosarcoma are rare and the application of available models is limited by low tumor incidence or long latency periods ^{12,46,48-50}.

Therefore, it was an object of the invention to elucidate the molecular mechanisms that contribute to the formation of rhabdomyosarcoma in order to provide a model system that is useful in developing therapies for this disease.

In the experiments of the invention, it was surprisingly found that mice carrying germline mutations in both the *p53* and *Fos* genes develop rhabdomyosarcoma with high penetrance and short latency. Re-expression of Fos in *p53; Fos* mutant rhabdomyosarcoma cells leads to increased spontaneous apoptosis suggesting that the disruption of the pro-apoptotic functions of Fos contributes to rhabdomyosarcoma development. These results suggest that mutations in the *p53* and *Fos* genes cooperate in the development of rhabdomyosarcoma and that *p53*; *Fos* mutant mice may be a potent mouse model for human rhabdomyosarcoma. They further indicate that Fos, in addition to its functions in oncogenic transformation and tumor progression, may also be a tumor suppressor.

Thus, the present invention relates to non-human mammals that carry germline mutations in both the *p53* and the *Fos* genes and their use as a model for rhabdomyosarcoma. (In the following, these transgenic animals are referred to as *"p53*/*Fos* double knockout" animals.) In related aspects, the invention relates to methods for generating *p53*/*Fos* double knockout animals as well as cell lines and tissues obtained from such animals.

### Detailed description of the invention

In the animals of the invention, both the *p53* and the *Fos* genes are mutated such that the animals do not express the functional gene products.

A "mutation" is a detectable change in the genetic material in the animal, which is transmitted to the animal's progeny. A mutation is usually a change in one or more deoxyribonucleotides, the modification being obtained by, for example, adding, deleting, inverting, or substituting for nucleotides.

Preferably, the genome of the transgenic non-human animal comprises one or more deletions in one or more exons of the genes and further comprises a heterologous selectable marker gene.

The term "knockout" characterizes a mutation of a nucleic acid sequence which significantly reduces, in particular suppresses or destroys expression of the biologically active encoded polypeptide. For example, *"Fos* knock-out animal" refers to an animal in which the expression Fos has been reduced or suppressed by the introduction of a recombinant nucleic acid molecule that disrupts at least a portion of the genomic DNA sequence encoding Fos.

In principle, knockout animals may have one or both copies of the gene sequence of interest disrupted. In the latter case, in which a homozygous disruption is present, the mutation is termed a "null" mutation. In the case where only one copy of the nucleic acid sequence of interest is disrupted, the knockout animal is termed a "heterozygous knockout animal". The *p53*/*Fos* double knockout animals of the present invention are preferably homozygous for the disruption of both the *p53* and the *Fos* genes.

Techniques for obtaining the transgenic animals of the invention are well known in the art; the techniques for introducing foreign DNA sequences into the mammalian germ line were originally developed in mice ⁵¹. One route of introducing foreign DNA into a germ line entails the direct microinjection of linear DNA molecules into a pronucleus of a fertilized one-cell egg. Microinjected eggs are subsequently transferred into the oviducts of pseudopregnant foster mothers and allowed to develop. About 25% of the progeny mice inherit one or more copies of the micro-injected DNA ⁵². Currently, the most frequently used techniques for generating chimeric and transgenic animals are based on genetically altered embryonic stem cells or embryonic germ cells. Techniques suitable for obtaining transgenic animals have been amply described, e.g. see in the manual by ^{53,54,55}. A suitable technique for obtaining completely ES cell derived transgenic non-human animals is described in WO 98/06834.

To generate the animals of the invention, in the first step, transgenic animals are generated that lack either p53 or Fos function. Such animals can be obtained by standard gene targeting methods as described above, preferably by using ES cells. In the next step, the transgenics are intercrossed to obtain *p53*/*Fos* double knockout mice.

Thus, in a further aspect, the invention relates to a method for producing a *p53*/*Fos* double knockout non-human animal comprising the steps
i. providing an embryonic stem cell from the relevant animal species comprising an intact *p53* gene and an embryonic stem cell from the relevant animal species comprising an intact *Fos* gene;
ii. providing a targeting vector capable of disrupting the *p53* gene and a targeting vector capable of disrupting the *Fos* gene upon homologous recombination;
iii. introducing said targeting vectors into said ES cells under conditions where the intact *p53* gene or the intact *Fos* gene, respectively, and the relevant targeting vector undergo homologous recombination to produce a mutant *p53* and a mutant *Fos* respectively,
iv. introducing the ES cells carrying a disrupted *p53* gene or *Fos* gene, respectively, into separate blastocysts;
v. implanting the blastocysts into the uterus of pseudopregnant females,
vi. delivering animals from said pseudopregnant female, selecting for heterozygous *p53*+/- and *Fos*+/- mice and breeding them to homozygosity,
vii. intercrossing homozygous *p53*-/- and *Fos*-/- animals, selecting for *p53*/*Fos* double knockout animals and breeding them.

A "targeting vector" is a vector comprising sequences that can be inserted into the gene to be disrupted, e.g., by homologous recombination.

The targeting vector generally has a 5' flanking region and a 3' flanking region homologous to segments of the gene of interest, surrounding a foreign DNA sequence to be inserted into the gene. For example, the foreign DNA sequence may encode a selectable marker, such as an antibiotics resistance gene. Examples for suitable selectable markers are the neomycin resistance gene (NEO) and the hygromycin β-phosphotransferase gene. The 5' flanking region and the 3'flanking region are homologous to regions within the gene surrounding the portion of the gene to be replaced with the unrelated DNA sequence. DNA comprising the targeting vector and the native gene of interest are contacted under conditions that favor homologous recombination. For example, the targeting vector and native gene sequence of interest can be used to transform embryonic stem (ES) cells, in which they can subsequently undergo homologous recombination.

Proper homologous recombination can be confirmed by Southern blot analysis of restriction endonuclease digested DNA using, as a probe, a non-disrupted region of the gene. Since the native gene will exhibit a restriction pattern different from that of the disrupted gene, the presence of a disrupted gene can be determined from the size of the restriction fragments that hybridize to the probe.

In an animal obtained in step vi, the extent of the contribution of the ES cells that contain the disrupted *p53* or the *Fos* gene to the somatic tissues of the transgenic can be determined visually by choosing animal strains for the source of the ES cells and blastocyst that have different coat colors.

In a preferred embodiment, the double knockout animals of the invention are mice. In other embodiments of this invention, the animals are rats, guinea pigs, rabbits, or dogs.

In another aspect, the present invention relates to the use of a *p53*/*Fos* double knockout animal, in particular a mouse, as a disease model for rhabdomyosarcoma.

In the *p53*/*Fos* double knockout mice, rhabdomyosarcomas develop at defined sites with short and reproducible latency.

In a further embodiment, the present invention relates to cells and tissues that carry mutations in both the p53 and the *Fos* genes. The cells can be primary cells or established cell lines obtained from the transgenic animals of the present invention according to routine methods, i.e. by isolating and disintegrating tissue from the *p53*/*Fos* double knockout animal and passaging the cells.

Such cells and tissues derived from the animals of the invention, in which the activity of p53 and Fos has been reduced or abolished, are useful in *in vitro* methods relating to rhabdomyosarcoma and the functional analysis of p53 and Fos in other cell types, e.g. osteoclasts and epithelial cells of the mammary gland or other organs.

In a further aspect, the invention relates to a method for determining whether a compound has therapeutic potential as a drug for the treatment of rhabdomyosarcoma and related disorders, wherein a candidate compound is administered to rhabdomyosarcoma bearing *p53*/*Fos* double knockout animals and the ability of the compound to inhibit tumor growth is correlated with its utility as a drug for the treatment of rhabdomyosarcoma.

The test compound can be deministered to the non-human *p53*/*Fos* double knockout animal in a variety of ways, e.g. orally, in a suitable formulation, by parenteral injection, subcutaneous, intramuscular, or intra-abdominal injection, infusion, ingestion, suppository administration, and skin-patch application. The effect of the compound on the tumor can be determined using methods well known to a person of ordinary skill in the art by analysing the incidence of tumor development, the growth of the tumor, the histopathological features of the tumor and the proliferation of the tumor cells.

In a method for screening compounds useful in rhabdomyosarcoma therapy, alternatively to administering the test compound to a *p53*/Fos double knockout animal, cells derived from such animal are incubated with the compound. A compound that has the ability to inhibit proliferation of the cells is a drug candidate and may be further assessed with regard to its therapeutic benefit for the treatment of rhabdomyosarcoma; e.g. by administering it to a *p53*/*Fos* double knockout animal as described above.

Cell lines derived from the *p53*/*Fos* double knockout animals are further useful to dissect the physiological and biochemical functions of p53 and Fos.

In a further embodiment, the present invention relates to cell lines derived from the *p53*/*Fos* double knockout animal into which a wildtype or modified p53 or a wildtype or modified Fos function has been reintroduced, i.e. that have been transfected with a wild type or modified p53 and/or Fos cDNA according to known methods. Such cell lines can be used for the analysis of p53 and Fos functions in biological processes, e.g. cell differentiation, proliferation, apoptosis, and invasive growth.

The experiments of the invention show that mice lacking both p53 and Fos develop high grade sarcomas of the orbital and facial region with almost complete penetrance at 6 months of age. Histologically, these tumors are pleomorphic, express markers of myogenic differentiation, and have a high proliferative activity resembling the embryonal variant of human rhabdomyosarcoma. *p53* homozygous and heterozygous mutant mice display enhanced tumor susceptibility and develop a broad range of tumors including rhabdomyosarcoma with low frequency ^{12,13,28}. The experiments of the invention suggest that the absence of Fos enhances tumorigenesis in *p53* mutant mice. This effect appears to be specific to the myogenic lineage since *p53*; *Fos* mutant mice otherwise displayed the same tumor spectrum as *p53* mutant mice, as only malignant lymphomas were observed in *p53*; *Fos* mutant mice up to 6 months of age. Although genetic alterations of the *TP53* gene are frequent in sporadic human rhabdomyosarcoma ^{7,8} and rhabdomyosarcoma is the most common sarcoma type in Li-Fraumeni syndrome ^{9,10}, a hereditary predisposition to cancer in patients who carry germ line mutations in the *TP53* gene ¹¹, rhabdomyosarcomas develop only with low incidence and long latency in *p53* heterozygous and homozygous mutant mice ^{12,13}. In the absence of Fos, the development of this tumor type was specifically enhanced and accelerated in both *p53* heterozygous and homozygous mutant mice. These results suggest that Fos, in addition to its oncogenic potential, may also be a tumor suppressor, at least in cooperation with p53, since tumors have not been observed in *Fos* single mutant mice ^{17,18}.

Previous studies have demonstrated that Fos is essential for osteoclast differentiation, acts as an oncogene *in vitro* and *in vivo,* mediates transforming signals, and controls invasive growth and angiogenesis during tumor progression ¹⁶. Furthermore, several observations suggest a role for Fos in myogenic differentiation. Fos expression is modulated during myoblast differentiation *in vitro* ²⁹ and ectopic Fos impairs skeletal muscle differentiation by inhibiting myoblast withdrawal from the cell cycle and fiber fusion during terminal differentiation ^{29,30}. Consistently, Fos negatively regulates the expression of the myogenic factors MyoD1 and myogenin ²⁹⁻³¹. The data that the formation of rhabdomyosarcomas in *p53* mutant mice is drastically enhanced in the absence of Fos suggest that Fos, although being dispensible for muscle development *in vivo,* may have a function in the suppression of tumorigenesis in the myogenic lineage. To study possible molecular mechanisms, Fos was re-introduced into *p53*; *Fos* mutant rhabdomyosarcoma cell lines. Ectopic Fos did not alter cell cycle progression but led to increased cell death of tumor cells suggesting that changes apoptosis contribute to rhabdomyosarcoma formation in *p53; Fos* mutant mice. The increase in apoptosis upon Fos re-expression is consistent with previous observations that Fos can have a pro-apoptotic function in various cell types including neurons, retinal photoreceptors, prostatic epithelial cells and B lymphocytes (reviewed in ¹⁶). It is conceivable that, in the absence of the pro-apoptotic p53 protein, the additional inactivation of Fos may render tumor precursor cells resistent to apoptosis. This may indicate that p53 and Fos act in two different apoptotic pathways, although Fos has been shown to be a target of transcriptional regulation by p53 ³², and that inactivation of two different apoptotic pathways is sufficient for tumor formation in the myogenic lineage.

In the experiments of the invention, a possible role for *Fos* inactivation in human rhabdomyosarcomas was investigated. PCR analysis of cDNAs prepared from 6 human rhabdomyosarcoma cells lines and 36 primary rhabdomyosarcomas of alveoloar and embryonal type indicated that *Fos* expression is not completely abolished in human rhabdomyosarcomas. Subsequent sequence analysis revealed wild-type *Fos* sequences in all tumors (data not shown). These results suggest that mutations in the coding regions of the *Fos* gene have no major role in the tumorigenesis of human rhabdomyosarcoma. However, it cannot be excluded that changes in Fos expression levels or mutations in Fos target genes might contribute to the development of human rhabdomyosarcomas.

### Brief description of the figures:

- Figure 1:: Rhabdomyosarcoma development in *p53*/*Fos* double knockout mice
- Figure 2:: Histological analysis of rhabdomyosarcomas in *p53*/*Fos* double knockout mice
- Fig. 3:: Histological analysis of muscle tissue in *p53*/*Fos* double knockout mice
- Fig. 4:: Fos-induced apoptosis in RMS cell lines

In the Examples, the following materials and methods were used:

### a) Mice

Generation, genotyping and phenotypic characterization of *p53* and *Fos* mutant mouse strains have been described previously ^{13,17}. *p53*/*Fos* double knockout mice were maintained on a mixed (129Sv x C57BL/6) genetic background. Mice were inspected once a week for morbidity and obvious tumor formation. Necropsies were performed on moribund mice.

### b) Histological analysis

Tissues were immersion-fixed in 4 % buffered formalin, embedded in paraffin, sectioned and stained with haematoxylin/eosin using standard histological techniques. For immunohistochemistry, antigen retrieval with citrate buffer was performed and tissue sections were incubated with primary antibody: desmin (clone Y-20; Santa Cruz), MyoD (clone 5.8A; DAKO), myogenin (F5D; DAKO), Ki-67 (clone TEC3; DAKO). Immunoreactivity was detected with biotinylated secondary antibodies and the biotin/streptavidin-horseradish peroxidase system (DAKO; Vector) using diaminobenzidine (DAB) as a chromogen.

### c) Generation of rhabdomyosarcoma cell lines, immunofluorescence staining

Tumor tissue were mechanically disaggregated and explanted into petri dishes. Pools of cells from three independently isolated tumors were established as cell lines and further characterized. Experiments were performed at passage 5-8 in culture. For all experiments cells were cultured in DMEM containing 10% fetal calf serum except for differentiation into myotubes which was performed in DMEM containing 5% horse serum.

Indirect immunofluorescence staining of cells was performed according to standard techniques using the following primary antibodies: desmin (clone Y-20; Santa Cruz), MyoD (clone 5.8A; DAKO) and α-actinin (Sigma).

### d) Retroviral gene transfer, proliferation and apoptosis assays

*p53* and *Fos* cDNAs were cloned into pBabe retroviral vectors containing an IRES-EGFP cassette to monitor expression. Cell lines at passage 6 were infected with viruses and selected with 2.5 ug/ml Puromycin according to standard procedures. Expression of *p53* and *Fos* was verified by Northern blot and Western blot analysis. For proliferation assays, 0.5x10⁵ cells were plated into 6-well dishes and counted every 24 hours for 6 consecutive days. Duplicates were counted for each cell line and time point. For apoptosis assays, cells were plated in duplicates at 0.5x10⁵ cells per 6-well dish and apoptosis was measured after 48hrs in culture using the Annexin-V cell death detection kit (Roche) according to the manufacturer's instructions. Representative results from at least two independent experiments for one cell line (pf1) are shown. Similar data were obtained with all three cell lines analyzed.

### Example 1

### p53/Fos double knockout mice develop rhabdomyosarcoma

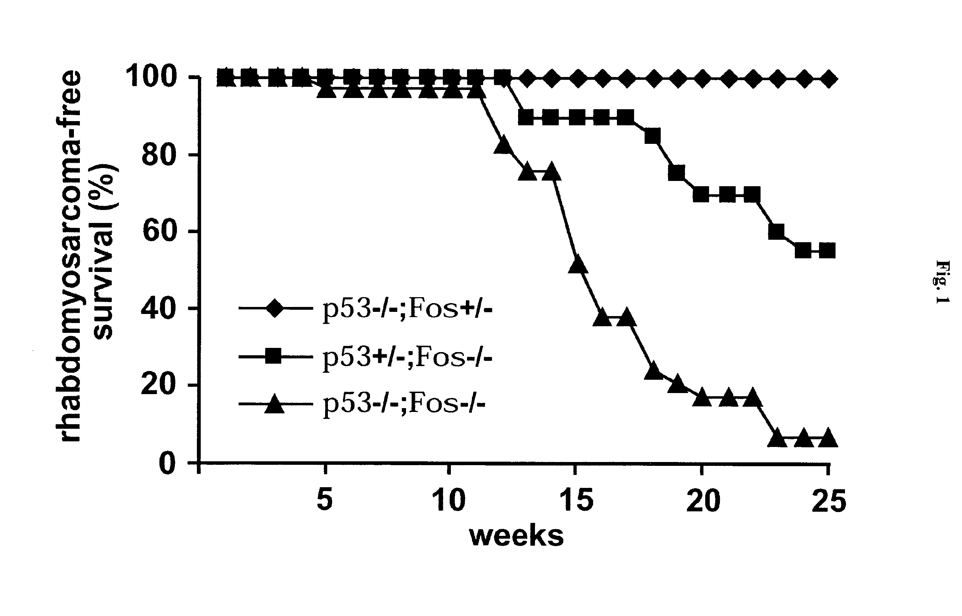

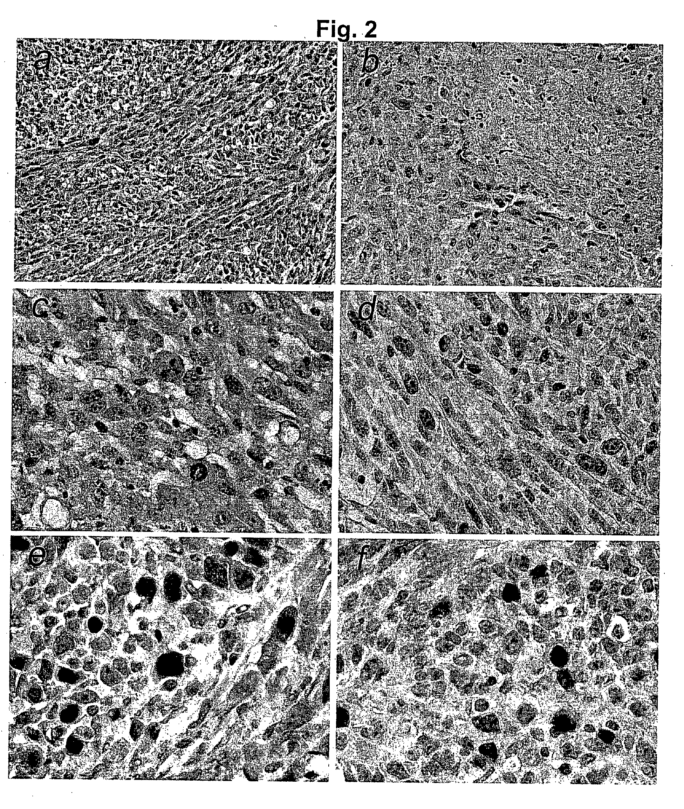

To study possible cooperative effects between germline mutations in the *p53* and *Fos* genes, *p53*-/- and *Fos*-/- mice were intercrossed to generate *p53*/*Fos* compound-mutant mice. These mice were obtained at almost Mendelian numbers and were phenotypically indistinguishable from *Fos*-/mice (data not shown). However, *p53*-/-; *Fos*-/- mice started to develop tumors of the facial and orbital regions at 10 weeks of age and tumor penetrance was 93 % at 25 weeks of age (Fig. 1). Tumors at these sites also grew in *p53*+/-; *Fos*-/- mice, although with lower penetrance, but were not observed in *p53*-/-, *Fos*+/- and *p53*+/-;*Fos*+/- mice (Fig. 1). Histological analysis of tumors revealed broad anastomosing fascicles of tumor cells with small areas of necrosis and invasive growth into adjacent muscle and fat (Fig.2a,b). Tumor cells were polygonal or elongated and had pleomorphic nuclei and abundant eosinophilic cytoplasm which displayed cross-striations in few neoplastic cells (Fig. 2c). Mitotic figures were numerous and staining for the cell cycle associated Ki-67 antigen revealed nuclear labeling in many tumor cells indicating high proliferative activity (Fig. 2d). Immunoreactivity for makers of myogenic differentiation including desmin, myogenin, and MyoD1 was observed in most tumor cells (Fig. 2e, f). Tumor cell morphology and maker profile were diagnostic of the embryonal type of rhabdomyosarcoma. These data suggest that *p53*/*Fos* compound-mutant mice develop embryonal rhabdomyosarcoma with high penetrance and short latency in the orbital and facial regions.

### Example 2

### Normal muscle development in p53/Fos double knockout mice

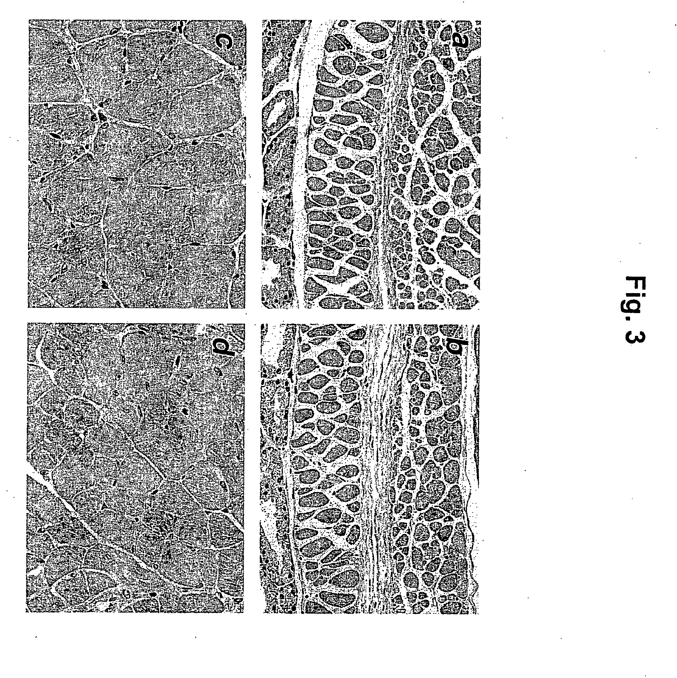

To analyse whether inactivation of the *p53* and *Fos* genes interferes with normal muscle development, histological analysis of orbital, facial and femoral muscles was performed in 6 to 10-week-old, tumor-free *p53*-/-; *Fos*-/- mice. No morphological abnormalities were found and comparable numbers of satellite cells and Ki-67 reactive cells were present in muscles derived from *p53*-/-; *Fos*-/- and *p53*+/-; *Fos*+/- mice (Fig. 3, data not shown). Therefore, muscle tumor development in *p53*/*Fos* compound-mutant mice does not reflect a general defect in muscle cell differentiation and increased numbers of progenitor cells.

### Example 3

### Fos regulates apoptosis in rhabdomyosarcoma cells in vitro

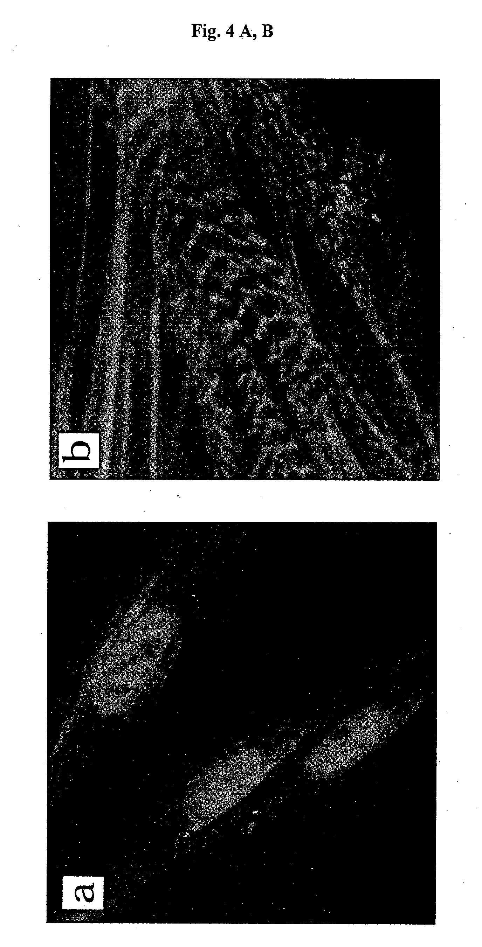

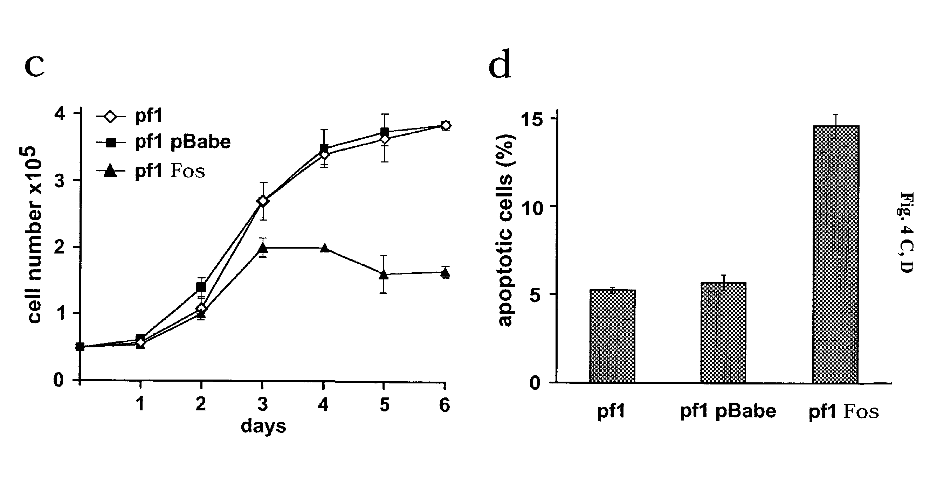

Enhanced rhabdomyosarcoma formation in *Fos*-deficient *p53* mutant mice suggested that Fos might regulate cell differentiation, proliferation or apoptosis in the myogenic lineage. To address and distinguish between these possibilities, three tumor cell lines were established. All cell lines showed characteristic myoblast morphology and expressed the muscle-specific markers MyoD and desmin (Fig. 4a). When grown at high density and in medium containing 5% horse serum, cells fused to form differentiated myotubes with cytoplasmic cross-striations, visualized by α-actinin staining (Fig 4b). These results provide additional evidence for the rhabdomyoblastic phenotype of the tumors in *p53*-/-;*Fos*-/- mice.

To study the molecular mechanisms underlying tumor development, the effects of re-expressing p53 and Fos in double mutant cell lines were analyzed by using retroviral gene transfer. Expression of p53 resulted in massive cell death and no cell lines could be established (data not shown). This is in agreement with p53 function to induce cell cycle arrest and apoptosis when over-expressed in cultured cells ²⁷. In contrast, the inventors were able to generate *p53*-/-;*Fos*-/- rhabdomyosarcoma cell lines re-expressing Fos at intermediate levels, as judged by Northern blot and Western blot analysis Surprisingly, cell lines expressing Fos grew significantly more slowly than non-infected or empty-vector controls, resulting in a 30-50% decrease in cell numbers after 6 days in culture (Fig. 4c, data not shown).

Whereas cell cycle analysis did not reveal significant changes in cell cycle progression (data not shown), a 2 to 3-fold increase in the number of apoptotic cells upon ectopic Fos expression was found (Fig. 4d). These data show that re-expression of Fos in *p53*/*Fos* -deficient muscle tumor cells leads to increased cell death without affecting cell proliferation and suggest that changes in apoptosis contribute to muscle tumor development in *p53*-/-;*Fos*-/- mice.

### References

- 1.: Dagher, R. & Helman, L. Rhabdomyosarcoma: an overview. *Oncologist* **4**, 34-44 (1999).
- 2.: Weiss, S.W.a.G., J.R. *Enzinger and Weiss's soft tissue tumors,* (Mosby, St. Louis, Missouri, USA, 2001).
- 3.: Barr, F.G. *et al.* Rearrangement of the PAX3 paired box gene in the paediatric solid tumour alveolar rhabdomyosarcoma. *Nat Genet* **3**, 113-7. (1993).
- 4.: Galili, N. *et al.* Fusion of a fork head domain gene to PAX3 in the solid tumour alveolar rhabdomyosarcoma. *Nat Genet* **5**, 230-5. (1993).
- 5.: Davis, R.J., D'Cruz, C.M., Lovell, M.A., Biegel, J.A. & Barr, F.G. Fusion of PAX7 to FKHR by the variant t(1;13)(p36;q14) translocation in alveolar rhabdomyosarcoma. *Cancer Res* **54**, 2869-72. (1994).
- 6.: Merlino, G. & Helman, L.J. Rhabdomyosarcoma--working out the pathways. *Oncogene* **18**, 5340-8. (1999).
- 7.: Mulligan, L.M., Matlashewski, G.J., Scrable, H.J. & Cavenee, W.K. Mechanisms of p53 loss in human sarcomas. *Proc Natl Acad Sci U S A* **87**, 5863-7. (1990).
- 8.: Felix, C.A. *et al.* Frequency and diversity of p53 mutations in childhood rhabdomyosarcoma. *Cancer Res* **52,** 2243-7. (1992).
- 9.: Li, F.P. *et al.* A cancer family syndrome in twenty-four kindreds. *Cancer Res* **48**, 5358-62. (1988).
- 10.: Garber, J.E. *et al.* Follow-up study of twenty-four families with LiFraumeni syndrome. *Cancer Res* **51,** 6094-7. (1991).
- 11.: Malkin, D. *et al.* Germ line p53 mutations in a familial syndrome of breast cancer, sarcomas, and other neoplasms. *Science* **250,** 1233-8. (1990).
- 12.: Harvey, M. *et al*. Spontaneous and carcinogen-induced tumorigenesis in p53-deficient mice. *Nat Genet* **5**, 225-9. (1993).
- 13.: Jacks, T. *et al.* Tumor spectrum analysis in p53-mutant mice. *Curr Biol* **4**, 1-7. (1994).
- 14.: Tamir, Y. & Bengal, E. p53 protein is activated during muscle differentiation and participates with MyoD in the transcription of muscle creatine kinase gene. *Oncogene* **17**, 347-56. (1998).
- 15.: Zhang, L. *et al.* Regulation of insulin-like growth factor II P3 promotor by p53: a potential mechanism for tumorigenesis. *Cancer Res* **56**, 1367-73. (1996).
- 16.: Jochum, W., Passegue, E. & Wagner, E.F. AP-1 in mouse development and tumorigenesis. *Oncogene* **20,** 2401-12. (2001).
- 17.: Wang, Z.Q. *et al.* Bone and haematopoietic defects in mice lacking c-fos. *Nature* **360**, 741-5. (1992).
- 18.: Johnson, R.S., Spiegelman, B.M. & Papaioannou, V. Pleiotropic effects of a null mutation in the c-fos proto-oncogene. *Cell* **71**, 577-86. (1992).
- 19.: Grigoriadis, A.E. *et al*. c-Fos: a key regulator of osteoclastmacrophage lineage determination and bone remodeling. *Science* **266,** 443-8 (1994).
- 20.: Finkel, M.P., Biskis, B.O. & Jinkins, P.B. Virus induction of osteosarcomas in mice. *Science* **151**, 698-701. (1966).
- 21.: Wang, Z.Q., Grigoriadis, A.E., Mohle-Steinlein, U. & Wagner, E.F. A novel target cell for c-fos-induced oncogenesis: development of chondrogenic tumours in embryonic stem cell chimeras. *Embo J* **10**, 2437-50 (1991).
- 22.: Grigoriadis, A.E., Schellander, K., Wang, Z.Q. & Wagner, E.F. Osteoblasts are target cells for transformation in c-fos transgenic mice. *J Cell Biol* **122**, 685-701 (1993).
- 23.: Hafezi, F. *et al.* The absence of c-fos prevents light-induced apoptotic cell death of photoreceptors in retinal degeneration in vivo. *Nat Med* **3**, 346-9 (1997).
- 24.: Schreiber, M., Baumann, B., Cotten, M., Angel, P. & Wagner, E.F. Fos is an essential component of the mammalian UV response. *Embo J* **14**, 5338-49. (1995).
- 25.: Saez, E. *et al*. c-fos is required for malignant progression of skin tumors. *Cell* **82**, 721-32 (1995).
- 26.: Orlandini, M., Marconcini, L., Ferruzzi, R. & Oliviero, S. Identification of a c-fos-induced gene that is related to the platelet- derived growth factor/vascular endothelial growth factor family [published erratum appears in Proc Natl Acad Sci U S A 1997 Feb 18;94(4):1603]. *Proc Natl Acad Sci U S A* **93**, 11675-80 (1996).
- 27.: Diller, L. *et al*. p53 functions as a cell cycle control protein in osteosarcomas. *Mol Cell Biol* **10**, 5772-81. (1990).
- 28.: Donehower, L.A. *et al*. Mice deficient for p53 are developmentally normal but susceptible to spontaneous tumours. *Nature* **356**, 215-21. (1992).
- 29.: Daury, L. *et al.* Opposing functions of ATF2 and Fos-like transcription factors in c-Jun- mediated myogenin expression and terminal differentiation of avian myoblasts. *Oncogene* **20**, 7998-8008. (2001).
- 30.: Lassar, A.B., Thayer, M.J., Overell, R.W. & Weintraub, H. Transformation by activated ras or fos prevents myogenesis by inhibiting expression of MyoD1. *Cell* **58**, 659-67. (1989).
- 31.: Pedraza-Alva, G., Zingg, J.M. & Jost, J.P. AP-1 binds to a putative cAMP response element of the MyoD1 promoter and negatively modulates MyoD1 expression in dividing myoblasts. *J Biol Chem* **269,** 6978-85. (1994).
- 32.: Elkeles, A. *et al*. The c-fos proto-oncogene is a target for transactivation by the p53 tumor suppressor. *Mol Cell Biol* **19**, 2594-600. (1999).
- 33.: Anderson, M.J., Shelton, G.D., Cavenee, W.K. & Arden, K.C. Embryonic expression of the tumor-associated PAX3-FKHR fusion protein interferes with the developmental functions of Pax3. *Proc Natl Acad Sci U S A* **98**, 1589-94. (2001).
- 34.: Bernasconi, M., Remppis, A., Fredericks, W.J., Rauscher, F.J., 3rd & Schafer, B.W. Induction of apoptosis in rhabdomyosarcoma cells through down- regulation of PAX proteins. *Proc Natl Acad Sci U S A* **93**, 13164-9. (1996).
- 35.: Ayyanathan, K. *et al.* Hormone-dependent tumor regression in vivo by an inducible transcriptional repressor directed at the PAX3-FKHR oncogene. *Cancer Res* **60**, 5803-14. (2000).
- 36.: Margue, C.M., Bernasconi, M., Barr, F.G. & Schafer, B.W. Transcriptional modulation of the anti-apoptotic protein BCL-XL by the paired box transcription factors PAX3 and PAX3/FKHR. *Oncogene* **19,** 2921-9. (2000).
- 37.: Epstein, J.A., Shapiro, D.N., Cheng, J., Lam, P.Y. & Maas, R.L. Pax3 modulates expression of the c-Met receptor during limb muscle development. *Proc Natl* Acad *Sci U S A* **93,** 4213-8. (1996).
- 38.: Ginsberg, J.P., Davis, R.J., Bennicelli, J.L., Nauta, L.E. & Barr, F.G. Up-regulation of MET but not neural cell adhesion molecule expression by the PAX3-FKHR fusion protein in alveolar rhabdomyosarcoma. *Cancer Res* **58**, 3542-6. (1998).
- 39.: Khan, J. *et al.* cDNA microarrays detect activation of a myogenic transcription program by the PAX3-FKHR fusion oncogene. *Proc Natl Acad Sci U S A* **96**, 13264-9. (1999).
- 40.: Khan, J. *et al.* Gene expression profiling of alveolar rhabdomyosarcoma with cDNA microarrays. *Cancer Res* **58,** 5009-13. (1998).
- 41.: El-Badry, O.M. *et al*. Insulin-like growth factor II acts as an autocrine growth and motility factor in human rhabdomyosarcoma tumors. *Cell Growth Differ* **1**, 325-31. (1990).
- 42.: Minniti, C.P., Tsokos, M., Newton, W.A., Jr. & Helman, L.J. Specific expression of insulin-like growth factor-II in rhabdomyosarcoma tumor cells. *Am J Clin Pathol* **101**, 198-203. (1994).
- 43.: Scrable, H.J., Witte, D.P., Lampkin, B.C. & Cavenee, W.K. Chromosomal localization of the human rhabdomyosarcoma locus by mitotic recombination mapping. *Nature* **329**, 645-7. (1987).
- 44.: Scrable, H. *et al.* Molecular differential pathology of rhabdomyosarcoma. *Genes Chromosomes Cancer* **1**, 23-35. (1989).
- 45.: Zhan, S., Shapiro, D.N. & Helman, L.J. Activation of an imprinted allele of the insulin-like growth factor II gene implicated in rhabdomyosarcoma. *J Clin Invest* **94,** 445-8. (1994).
- 46.: Hahn, H. *et al.* Rhabdomyosarcomas and radiation hypersensitivity in a mouse model of Gorlin syndrome. *Nat Med* **4**, 619-22. (1998).
- 47.: Hahn, H. *et al.* Patched target lgf2 is indispensable for the formation of medulloblastoma and rhabdomyosarcoma. *J Biol Chem* **275**, 28341-4. (2000).
- 48.: Teitz, T., Chang, J.C., Kitamura, M., Yen, T.S. & Kan, Y.W. Rhabdomyosarcoma arising in transgenic mice harboring the beta- globin locus control region fused with simian virus 40 large T antigen gene. *Proc Natl Acad Sci U S A* **90,** 2910-4. (1993).
- 49.: Takayama, H. *et al.* Diverse tumorigenesis associated with aberrant development in mice overexpressing hepatocyte growth factor/scatter factor. *Proc Natl Acad Sci U S A* **94**, 701-6. (1997).
- 50.: Vogel, K.S. *et al.* Mouse tumor model for neurofibromatosis type 1. *Science* **286,** 2176-9. (1999).
- 51.: Hogan et al., eds; *Manipulating the Mouse Embryo*, 2d ed., Cold Spring Harbor Press, (1994)
- 52.: Brinster, R.L. et al., Somatic Expression of Herpes Thymidine lunase in Mice following Injection of a Fusion Gene into Eggs. *Cell* 27: 223-231 (1981).
- 53.: Joyner et al., eds; *Gene Targeting. A Practical Approach.* Oxford University Press (1995)
- 54.: Müller, U., Ten years of gene targeting: targeted mouse mutants, from vector design to phenotype analysis. *Mechanisms of Development* 82:3-21 (1999)
- 52.: Brinster, R.L. et al., Somatic Expression of Herpes Thymidine lunase in Mice following Injection of a Fusion Gene into Eggs. *Cell* 27: 223-231 (1981).
- 53.: Joyner et al., eds; *Gene Targeting. A Practical Approach.* Oxford University Press (1995)
- 54.: Müller, U., Ten years of gene targeting: targeted mouse mutants, from vector design to phenotype analysis. *Mechanisms of Development* 82:3-21 (1999)
- 55.: Plump, A.S. et al., Severe Hypercholesterolemia and Atherosclerosis in Apolipoprotein E-Deficient Mice Created by Homologous Recombination in ES Cells. *Cell* 71:343-353 (1992).

## Claims

**1.** A non-human mammal that carries germline mutations in both the *p53* and the *Fos* genes.

**2.** The animal of claim 1 which is a mouse.

**3.** A method for producing the *p53*/*Fos* double knockout non-human animal of claim 1 comprising the steps
i. providing an embryonic stem cell from the relevant animal species comprising an intact *p53* gene and an embryonic stem cell from the relevant animal species comprising an intact *Fos* gene,
ii. providing a targeting vector capable of disrupting the *p53* gene and a targeting vector capable of disrupting the *Fos* gene upon homologous recombination,
iii. introducing said targeting vectors into said ES cells under conditions where the intact *p53* gene or the intact *Fos* gene, respectively, and the relevant targeting vector undergo homologous recombination to produce a mutant *p53* and a mutant *Fos* gene, respectively,
iv. introducing the ES cells carrying a disrupted *p53* gene or a disrupted *Fos* gene, respectively, into separate blastocysts,
v. implanting the blastocysts into the uterus of pseudopregnant females,
vi. delivering animals from said pseudopregnant female, selecting for heterozygous *p53*+/- and *Fos*+/- mice and breeding them to homozygosity,
vii. intercrossing homozygous *p53*-/- and *Fos*-/- animals, selecting for *p53*/*Fos* double knockout animals and breeding them.

**3.** The method of claim 3, wherein the animal is a mouse.

**4.** The use of the *p53*/*Fos* double knockout animal of claim 1 of as a disease model for rhabdomyosarcoma.

**5.** The use of claim 4 wherein the animal is a mouse.

**6.** A method for determining whether a compound has therapeutic potential as a drug for the treatment of rhabdomyosarcoma, wherein a test compound is administered to a rhabdomyosarcoma bearing *p53*/*Fos* double knockout animal and the ability of the compound to inhibit tumor growth is correlated with its utility as a drug for the treatment of rhabdomyosarcoma.

**7.** A cell line derived from a *p53*/*Fos* double knockout animal of claim 1 or 2.

**8.** A method for determining whether a compound has therapeutic potential as a drug for the treatment of rhabdomyosarcoma, comprising incubating the cells of claim 7 with a test compound and correlating the ability of the compound to inhibit proliferation of the cells with its utility as a drug for the treatment of rhabdomyosarcoma.

**9.** The cell line of claim 7, transfected with a wild type or modified *p53* and/or a wildtype or modified *Fos* cDNA.
